# EUROPEAN PATENT SPECIFICATION

(11) **EP 3 426 043 B1**
(45) Date of publication and mention of the grant of the patent: **21.06.2023**
(21) Application number: 17763805.3
(22) Date of filing: 03.03.2017
(51) Int. Cl.: A61K 36/18, A61K 36/076, A61K 36/481, A61K 36/536, A61K 36/756, A61P 43/00

(54) **HERBAL FORMULATION TO RAISE THE HUMAN BODY'S PH LEVEL FROM ONE THAT IS ACIDIC TO ONE THAT IS MORE ALKALINE**
KRÄUTERFORMULIERUNG ZUR ERHÖHUNG DES PH-WERTS DES MENSCHLICHEN KÖRPERS VON SAUER AUF BASISCH
FORMULATION À BASE DE PLANTES POUR AUGMENTER LE PH DU CORPS HUMAIN ET LE FAIRE PASSER D'UN PH ACIDE À PH PLUS ALCALIN

(30) Priority: 08.03.2016 US 201662304945 P
(43) Date of publication of application: 16.01.2019
(73) Proprietor: Cheng Lab&Company, LLC, Houston, TX 77074-3546 (US); Cheng, Yin, Bong, Houston, TX 77074-3546 (US)
(72) Inventor: CHENG, Yin, Bong, Houston, TX 77074-3546 (US)
(74) Representative: Charrier Rapp & Liebau
(86) International application number: PCT/US2017/020806
(87) International publication number: WO 2017/155834

(56) References cited:
- US-A1- 2009 104 295
- US-A1- 2014 128 421
- US-A1- 2015 297 652
- DATABASE WPI Week 201536 Thomson Scientific, London, GB; AN 2015-183721 XP002793942, & CN 104 288 214 A (UNIV CHONGQING NORMAL) 21 January 2015 (2015-01-21)
- DATABASE WPI Week 201034 Thomson Scientific, London, GB; AN 2010-E97005 XP002793943, & CN 101 697 982 A (BEIJING HERUN CHUANGXIN PHARM TECHN DEV) 28 April 2010 (2010-04-28)
- DATABASE WPI Week 200956 Thomson Scientific, London, GB; AN 2009-M36188 XP002793944, & CN 101 491 555 A (BEIJING HERUN CHUANGXIN PHARM TECHN DEV) 29 July 2009 (2009-07-29)
- DATABASE WPI Week 201545 Thomson Scientific, London, GB; AN 2015-34198E XP002794111, & CN 104 491 702 A (LI Y) 8 April 2015 (2015-04-08)
- DATABASE WPI Week 201626 Thomson Scientific, London, GB; AN 2016-12680X XP002794112, & CN 105 311 440 A (HENAN YONGCHUAN PHARM TECHNOLOGY CO LTD) 10 February 2016 (2016-02-10)
- DATABASE WPI Week 201510 Thomson Scientific, London, GB; AN 2015-090363 XP002794113, & CN 104 189 859 A (LI Y) 10 December 2014 (2014-12-10)
- DATABASE WPI Week 200525 Thomson Scientific, London, GB; AN 2005-234061 XP002794114, & CN 1 557 380 A (QUE F) 29 December 2004 (2004-12-29)
- DATABASE WPI Week 200382 Thomson Scientific, London, GB; AN 2003-878238 XP002794115, & CN 1 440 769 A (GUO W) 10 September 2003 (2003-09-10)
- DATABASE WPI Week 201545 Thomson Scientific, London, GB; AN 2015-34198E XP002794117, & CN 104 491 702 A (LI Y) 8 April 2015 (2015-04-08)
- 'Body Alkaline Formula' MERCHANTS OF THE WORLD, [Online] 2010, page 1, XP055419753 Retrieved from the Internet: <URL:https://merchantsoftheworldstore.com/b ody-alkaline-formula>

## Description

### Technical Field of the Invention

The present invention relates to the field of herbal supplements to improve overall health.

Document CN 104 288 214 A (UNIV CHONGQING NORMAL) concerns a herbal and medicinal composition in the treatment of *diarrhea,* comprising *Stachys sieboldii, Astragalus membranaceus, Ginseng* and *Elecampanae* having the pH of 9-10

Document CN 101 697 982 A (BEIJING HERUN CHUANGXIN PHARM TECHN DEV) relates to the separation of Camptotheka acumintaa total alkaloid by adjusting pH of Camptotheka acuminata extract solution, adjusting at pH 1-7, filtering, adding the filtrate to cation exchange resin column, eluting with water and soaking resin column with salt solution.

Document CN 101 491 555 A (BEIJING HERUN CHUANGXIN PHARM TECHN DEV) discloses the separation of total alkaloids from Camptotheca acuminata extract by a process comprising adjusting pH of the extract at 1-7

Document CN 104 491 702 A (LI Y) concerns a traditional chinese medicine (TCM) for use in treating choriocarcinoma, containing *Astragalus membranaceus, Camptotheca acuminata* and *Poria* cocos.

Document CN 105 311 440 A (HENAN YONGCHUAN PHARM TECHNOLOGY CO LTD) discloses a traditional chinese medicine tea used for increasing height, comprising *Astragalus membranaceus* and *Camptotheca acuminata.*

Document CN 104 189 859 A (LI Y) relates to a traditional chinese medicine composition for treating lung cancer comprising *Astragalus* and *Camptotheca* fruit.

Document CN 1 557 380 A (QUE F) discloses a chinese medicine preparation for treating cancer, comprising common *Camptotheca* seed and *Astragalus* root.

Document CN 1 440 769 A (GUO W) reveals a chinese herbal medicine for treating hemopathy, e.g., anemia, comprising *Astragalus* root and common *Camptotheca* leaf.

The present invention discloses an herbal formula that adjusts the pH balance of the human body's fluids to lower acidity in minimal time.

### Background of the Invention

The overall health benefits of maintaining slightly alkaline body fluids are well known. Changing the pH level to one that is slightly alkaline helps the body safeguard itself against illness and numerous life threatening diseases. Unfortunately, popular foods and beverages that are widely consumed adversely affect the body's pH level. Coffee, soda, alcohol, dairy products, red meat, juices, sugar, and fried foods are highly acidic as are many ingredients used in restaurants or prepared/boxed foods such as preservatives, additives, and coloring agents.

Natural and whole foods such as raw vegetables, salads, fresh fruit, and whole grains can counteract excessive acidity levels attributed to poor dietary choices. In the past, in order to raise the alkalinity of body fluids, health professionals recommended an alkaline diet which includes anise seeds, barley grass, burdock roots, caraway seeds, dill seeds, fennel seed, mustard seeds, wheat grass, bilberry, sea products like kelp, and dulse, as well as herbal complexes which includes rose hip, turmeric root, horseradish, dandelion ginger, parsley, garlic, and white willow etc. Few people, however, eat sufficient quantities of such foods or can reasonably implement and maintain such a diet long enough to raise the alkalinity of their body fluids in the necessary amount of time it takes, which can be months or years.

These and other advantages of the present invention will become apparent from the following description.

### Summary of the Invention

According to the present invention, a herbal formulation which raises the alkalinity of the human body in a short period of time is disclosed. The herbal formulation include the three ingredients of Camptotheca, poria cocos and astragalus membranacaus. The major ingredient in the formulation is Camptotheca. Although the formulation may consist entirely of Camptotheca, poria cocos and astragalus membranacaus, it preferably further includes ostrea vivularis gould, prunella vulgaris, bombyx mori and phellodendron amurense Rupr.

While the herbal formulation for use to increase the alkalinity of the human body in accordance with the present invention may be a mixture containing Camptotheca, poria cocos and astragalus membranacaus and all or some of the other above ingredients, it is most preferred that the herbal formulation include the three ingredients of Camptotheca, poria cocos and astragalus membranacaus.

### Detailed Description of the Invention

According to the present invention, an edible herbal formulation which raises the alkalinity of the human body in a short period of time is disclosed. The major ingredient in the formulation is Camptotheca which may be present in the formulation in the amount in the range from 1% to 99% by weight. The genus Camptotheca is included in the dogwood family Cornaceae where it has two species, namely, Camptotheca acuminata decne and Camptotheca Lowreyana. The Camptotheca ingredients in the formulation may include seeds, leaves, bark, stems and roots. It should be noted that chemical ingredients from Camptotheca containing alkaloid camptothecin and several chemical derivatives of camptothecin are presently being studied for use as drugs for cancer treatment, including irinotecan, topotecan, and rubitecan. Camptotheca also contains the chemical compounds trifolin and hyperoside.

While the formulation may consist entirely of Camptotheca, poria cocos and astragalus membranacaus to effect the desired increase in the alkalinity, it is preferred that the formulation include other beneficial ingredients in quantities which may range from 1 % to 20% by weight. Those ingredients are ostrea vivularis gould, prunella vulgaris, bombyx mori and phellodendron amurense Rupr.

Poria cocos contains β - pachyman, pachymic acid, tuonulosic acid, 3 β. hydroxylanasta, etc. It has diuretic, antibacterial and antacid effects.

Astragalus membranacaus contains choline, betaine, quercetin, sitostero, etc. Its beneficial effects are as pH raiser, antacid, vascular dilation, and pulse reducer

Ostrea vivularis gould contains 80% - 95% calcium carbonate and acts as an antacid and an antiperspiration agent.

Prunella vulgaris contains oleanolic acid, ursolic acid, rutin, hyper side. Its beneficial effects are as antihypertension agent, antibacterial, and vascular dilation.

Bombyx mori contains phytol, β-sitostorol, cholesterol, ergesterol, tetracosanol, and lupeol and they remove wind and dampness (toxical invisible gas matter) and are anti-itching.

Phellodendron amurense Rupr contains barberine, jatrorrhizine, magnoflorine, phellodendrone, obacurone, obacunoni acid, etc. Its beneficial effects are to act as an antibacterial and to lower blood pressure. Phellodendron, however, has a bitter taste that is a disadvantage when used for diets.

While the herbal formulation for use to increase the alkalinity of the human body in accordance with the present invention may be a mixture containing Camptotheca and all or some of the above listed ingredients, it is most preferred that the herbal formulation include the three ingredients of Camptotheca in the range of 10% to 90% by weight, poria cocos in the range of 5% to 20% by weight and astragalus membranacaus in the range of 5% to 20% by weight. In that combination poria cocos adds to the beneficial effects of Camptotheca in increasing the alkalinity of the human body and astragalus membranacaus substantially reduces, if not eliminates, the side effects of Camptotheca.

The following examples further illustrate the invention.

### Preparation of Herbal Formulations

### Sample A for Comparison

One hundred percent (100%) Camptotheca powder was prepared by taking fruits, leaves, seeds and branches from Camptotheca grown in the inventors own herb garden, drying them in 250° F for four hours and grinding them to powder.

### Sample B

Dry Camptotheca powder from sample A was mixed with prunella vulgaris (Chinese Herbal tea extract, Ming-Tong 1164 which is available commercially). The amount of Camptotheca powder was 80% by weight and the balance 20% by weight was prunella vulgaris.

### Sample C

Dry Camptotheca powder from sample A was mixed with astragalus membranaceus (concentrated extract from Ming-Tong 1074) and poria cocos in dry powder form of natural poria cocos. The amount of Camptotheca powder was 75% by weight, the amount of astragalus membranaceus was 15% by weight and the balance 10% by weight was poria cocos.

### Treatment Example 1 for Comparison

A 66 year-old Asian woman who was in good general health took a Quantum Resonance Magnetic Analyzer test. The test showed a pH score of 3.194 which is within the normal score range of 3.156 to 3.694. Out of 206 total test items, 25 items were out of normal scope. Then, she took herbal formulation from Sample A daily at a rate of 1 gram per day for a month. After taking herbal formulation from Sample A for a month, she was retested. The basic physical quality (pH) showed an elevated pH score of 3.267 and the out-of-norm items were reduced from 25 to 20.

### Treatment Example 2

A 75 year old Asian woman who has been suffering for the last 20 years with diabetes had low back pain due to a ruptured disc. She took a Quantum Resonance Magnetic Analyzer test which showed a pH score of 3.053 and a health scope having 32 items off scale. She was put on a regimen of 1000mg of herbal formulation from Sample B taken twice per day for twelve days while undergoing daily acupuncture treatment for low back pain. Twelve days later, she was retested. The basic physical quality (pH) showed an elevated pH score of 3.199 which is within the normal range and the out-of-norm items were reduced from 32 to 23. The test showed a much improved health overall. Further, the low back pain was completely gone.

### Treatment Example 3

A 63 year old Asian man complained of cervical hyperplasia. He took a Quantum Resonance Magnetic Analyzer test which showed a pH score of 2.962 and a health scope having 33 items off scale. He rejected the recommendation of taking herbal formulation of Sample B; instead, he wanted to pursue his own way to improve the alkalinity of his body by eating alkaline forming diet foods such as oranges, lemons, vegetables, etc. Three months later he took another Quantum Resonance Magnetic Analyzer test that showed a pH score of 2.946 which was lower than the pH score of the first test. It also showed a scope having 37 items off the normal range. He was then put on a regimen of 3000mg of herbal formulation per day for experimental use. One month later he took a third Quantum Resonance Magnetic Analyzer test which showed a pH score of 3.125 which was still lower than 3.156. It also showed an improved health scope having 28 items off scale. Another month later, he took a fourth Quantum Resonance Magnetic Analyzer test which showed a pH score of 3.229 which is between 3.156 and 3.694. The out of scale items dropped down to 21, an indication of great improvement in overall health scope.

### Treatment Example 4

A 64 year old male patient with a family history of Alzheimer's and colon cancer and a personal history of sports injuries, sciatica, arthritis, CTE (Chronic Traumatic Encephalopathy) with Parkinson syndrome was treated with the herbal formulation of the present invention over a period of several months. On March 18, 2016, he took a Quantum Resonance Magnetic Analyzer test which showed a pH score of 3.101 which is outside the normal range and a health scope having 33 items outside the normal range.

Starting on March 18, 2016 and continuing thereafter on a daily basis, the patient took 1 gram of herbal formulation comprising camptotheca (75% by weight), astragalus membranaceus (15% by weight) and poria cocos (10% by weight twice a day. Thereafter, the patient took the Quantum Resonance Magnetic Analyzer test on May 6, 2016, July 8, 2016, September 8, 2016, and November 7, 2016. During the testing period of March 18, 2016 to November 7, 2016, results indicate improvement of patient's pH index to Normal range from 3.101 on March 8, 2016, to 3.188 on May 6, 2016, to 3.459 on July 8, 2016, to 3.423 on September 8, 2016, and then to 3.209 on November 7, 2016. Further, the total number of out of Normal health systems decrease from 33 on March 8, 2016, to 23 on May 6, 2016, to 18 on July 8, 2016 and then to 10 on November 7, 2016. Twenty three health systems showed improvement to Normal range during the subject testing period testing period. That is an indication that by improving the acidity level of body fluids to Normal range, additional health systems will show improvement as well. The results of each test and the improvement in the health systems is shown in detail in Table 1.

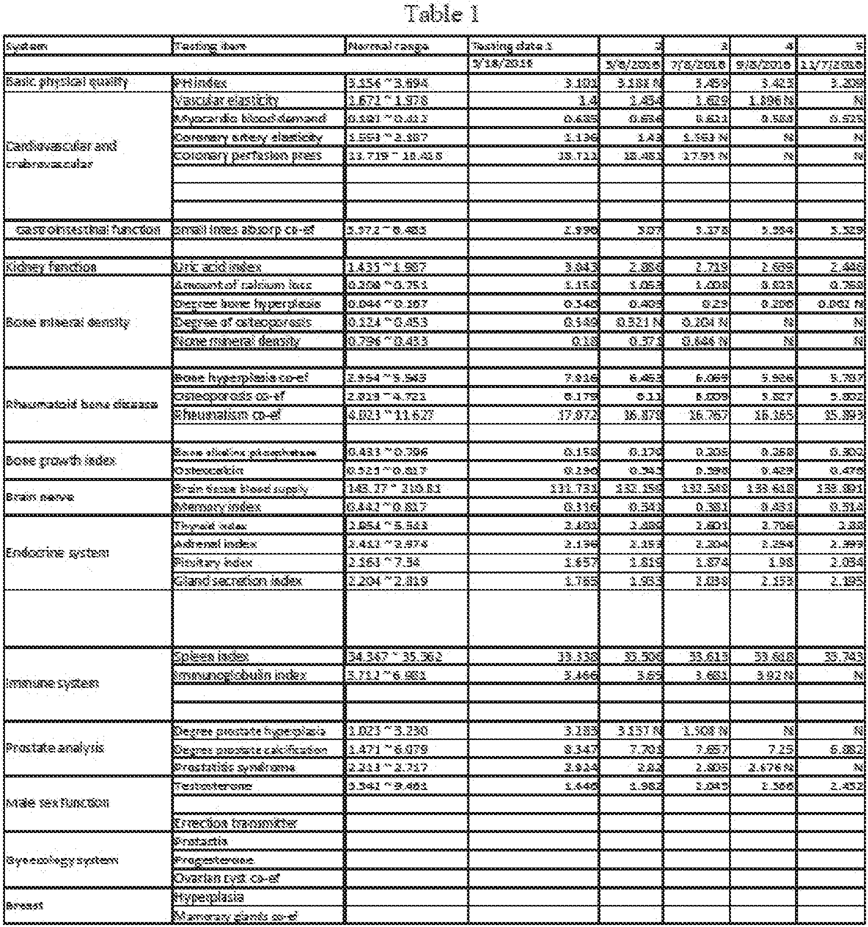

Referring to Table 1, during the testing period, the following improvements took place in the health systems of the patient.
A. Four items in the Cardiovascular System improved to Normal. This indicates that when pH index improves, cardiovascular and cerebrovascular systems can be reversed to healthy conditions.
B. The gastrointestinal function of small intestine absorption coefficient improves from 2.996 to 3.529, which indicates improvement to a near Normal range (3.572 ∼ 6.483). This indicates that when pH index improves, gastrointestinal functions improve as well.
C. The uric acid index decreased from 3.043, as tested on March 18, 2016, to 2.446, as tested on November 7, 2016. The Normal range is 1.435 - 1.987. This improvement indicates that when pH index improves, the uric acid index improves as well.
D. Ten items related to bone mineral density, bone growth, and all the bone related items, including bone hyperplasia, degree of osteoporosis, bone alkaline phosphatase tested out of Normal range. During the testing period, as pH levels improved, bone related health also greatly improved. This improvement indicates that when pH index improves, bone health improves as well.
E. The important brain nerve system including the cerebroblood supply and memory index improves from the first test of March 18, 2016 at 131.731 (normal 143.27 - 210.81) and 0.316 (normal 0.442 - 0.817) to the fifth test of November 7, 2016 at 133.891 and 0.514, respectively. This indicates that changing the acidity levels of body fluids to more alkaline improves memory and brain functions.
F. The endocrine system, which initially shows four items out of Normal range, including thyroid, adrenal, pituitary and gland secretion, indicates all four areas have improved during testing period.
G. On the first test ob March 18, 2016, the immune system, including spleen index and immunoglobulin index, were out of Normal range at 1 33.338 (NR 34.367 - 35.362) and 3.466 (NR 3.712 - 6.981). When the fourth test was taken September 8, 2016, both improved to Normal range 33.618 and 3.92. This indicates that a pH index in the Normal range means the immune system will stay in healthy condition.
H. Male prostate organ, including degree of prostatic calcification, degree of prostatic hyperplasia and prostatitis syndrome, on the first test of March 18, 2016 were 8.347 (NR 1.471 - 6.079), 3.283 (NR 1.023 - 3.230) and 2.924 (NR 2.213 ∼ 2.717). In the fourth test of September 8, 2016 the results were 7.25, 1.505(NR), and 2.676 (NR). This indicates prostate health is improved when pH balance is in Normal range.
I. The first test of March 18, 2016, in male sex function showed testosterone to be out of Normal range 1.646 (NR 3.342 - 9.461). The fifth test on November 7, 2016 showed an improved testosterone index of 2.452. This indicates sex function is improved when pH balance is in Normal range.

As shown on Table 1, the first test of March 18, 2016 indicates that 33 health systems were out of Normal range. Only about eight months later, the results of the fifth test on November 7, 2016, On Test 5, indicate only ten items were out of Normal range. It should be noted that during this period, the patient did not take any additional or prescribed medications (from conventional physicians). This indicates that by improving the acidity level of body fluids to Normal range, additional health systems will show improvement as well.

### Treatment Example 5

A 67 year old male patient with a left disintegrated hip bone had a scheduled surgery. Because he suffered from bronchiolitis obliterans organizing pneumonia (BOOP), LIPS, asthma, and cough he needed clearance for the surgery from his primary care physician, a pulmonologist, a cardiologist and one other doctor. He was taking 22 prescribed medications including Prednisone and Hydrocodone.

On October 14, 2016 , he took a Quantum Resonance Magnetic Analyzer test which showed a pH score of 3.15 (an indication of acidity and outside the normal range) and a health scope having 42 additional health systems to be outside of normal range. Although his pH index was just below the normal range, starting on October 14, 2016 and continuing thereafter on a daily basis, the patient took 1 gram of herbal formulation comprising camptotheca (75% by weight), astragalus membranaceus (15% by weight) and poria cocos (10% by weight) twice a day.

Further, the subj ect began undergoing acupuncture treatment and after the second week of the acupuncture treatment, he stopped taking all the medication prescribed by his doctors. He only took the herbal formulation of the present invention.

Thereafter, the patient took the Quantum Resonance Magnetic Analyzer test on December 13, 2016 and the patient's pH index reached normal. The rest of test results show great improvement. The total number of out of normal health systems decreased from 43 to 31. His pulmonary system improved dramatically. The asthma, BOOP, LIPS, and cough completely healed. The cardiovascular system improved greatly. This indicates that when pH index improves, cardiovascular and cerebrovascular systems can be reversed to healthy conditions. The results of each test and the improvement in the health systems is shown in detail in Table 2.

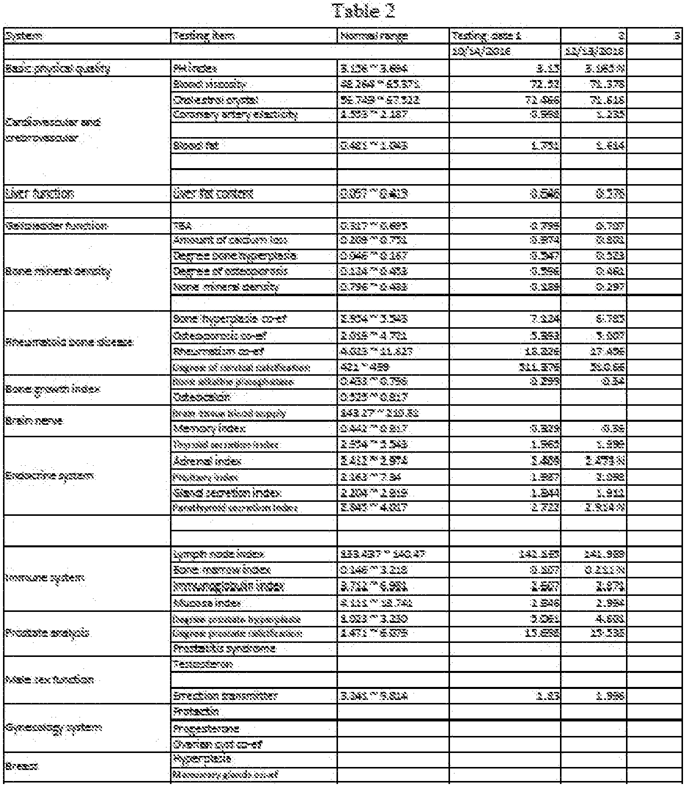

Referring to Table 2, during the testing period, the following improvements took place in the health systems of the patient.
A. During this period, due to the fact that the subject's pH index was raised into normal range, the immune system and endocrine system all improved thereby contributing to the improvement of the pulmonary system. This is an indication that by improving the acidity level of body fluids to normal range, additional systems will show improvement as well.
B. Fourteen (14) items related to bone mineral density, rheumatoid bone disease, bone growth index, and other bone related issues were off normal in the first test of October 14, 2016. As pH level improved, bone related off normal items were decreased to only 8, as indicated by the test of December 13, 2016.
C. Male prostate organ and male sex function system showed improvement between the two tests. This indicates prostate health and male sex function improved when pH balance is in normal range.

As shown on Table 2, the first test of October 14, 2016 indicates that 43 health systems were out of normal range. On the second test of December 13, 2016, only two months later, the patient test results indicated 31 items are out of normal range. It should be noted that during this period, the patient eliminated his prescribed medications except two Motrin and one aspirin a day, as needed. This indicates that by improving the acidity level of body fluids to normal range, additional health systems will show improvement as well.

### Treatment Example 6

A 57 year old male patient with a personal history of frequent headaches, recurring yeast infection, chronic fatigue, acid reflux was treated with the herbal formulation of the present invention over a period of several months. A few months before he was subjected to the treatment, he had a cold and took three courses of antibiotic as treatment.

On February 19, 2016, he took a Quantum Resonance Magnetic Analyzer test which showed a pH score of 2.821 which is quite outside the normal range and a health scope having 38 items outside the normal range.

Starting on February 19, 2016 and continuing thereafter on a daily basis, the patient took 1 gram of herbal formulation comprising camptotheca (75% by weight), astragalus membranaceus (15% by weight) and poria cocos (10% by weight twice a day. Thereafter, the patient took the Quantum Resonance Magnetic Analyzer test on May 4, 2016, August 8, 2016, and December 9, 2016. Referring to Table 3 which is a tabulation of the test results of those dates, during the testing period, results indicate improvement of patient's pH index and took him ten months to reach a pH index of 3.196 which is within the normal range, as shown in the results of the test of December 9, 2016.

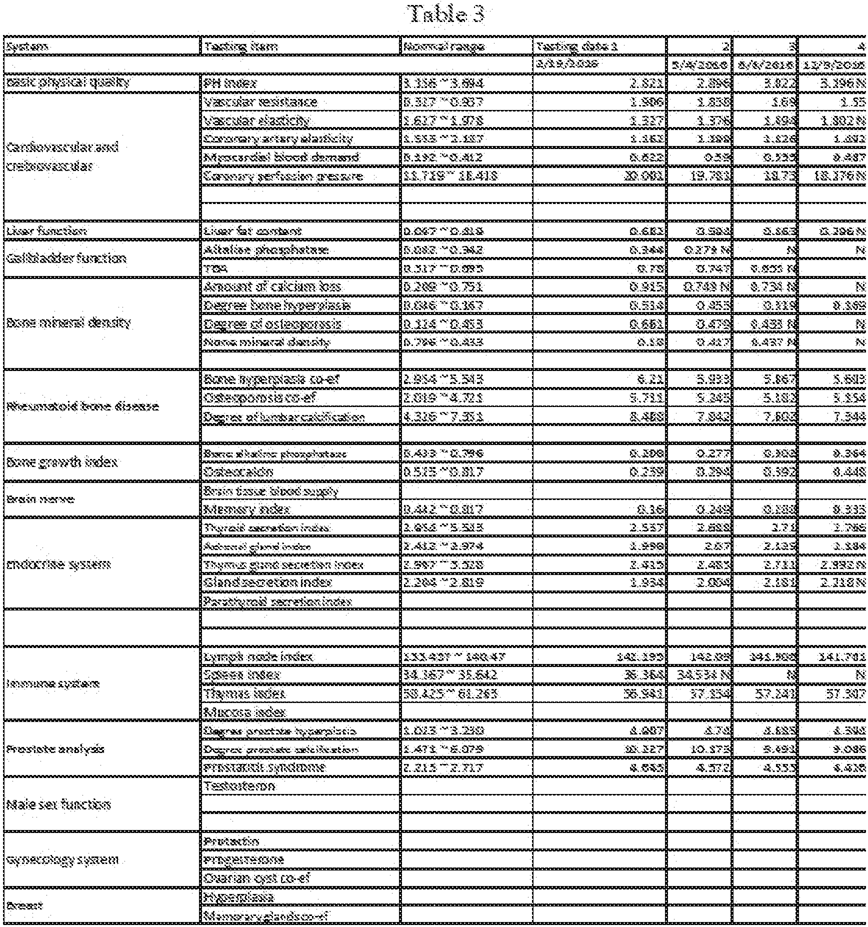

Still referring to Table 3, during the testing period, the following improvements took place in the health systems of the patient.
A. During this period all the systems show steady improvement including cardiovascular, cerebrovascular, liver fat, gallbladder function such as alkaline phosphatase, ALP and TBA ( serum total bile acid) , bone density and bone growth index.
B. During this period, one of the notable improvements is his Brain Memory Index. In his first test it was 0.16, way too low from the normal of 0.444. He admitted that his brain is foggy, he is forgetful, difficult to concentrate. At the 5th test, it shows his memory index is 0.333, getting close to the Normal range. He also recognized his thought as being clear and fresh.
C. During this ten months period, his endocrine system, immune system and the prostate health were also improved greatly.

As shown on Table 3, the first test of February 19, 2016, indicated that 38 health items were out of normal range. Ten months later, on December 9, 2016, the patient's test result indicated that only 16 health items were outside the normal range. It should be noted that during that period, the patient did not take any prescribed medications from conventional doctors. This indicates that by improving the acidity level of body fluids to normal range, additional health systems will show improvement as well.

## Claims

1. An herbal formulation for use in increasing the alkalinity of the human body, comprising:
Camptotheca;
poria cocos; and
astragalus membranacaus.

2. An herbal formulation for use according to claim 1, further including ostrea vivularis gould.

3. An herbal formulation for use according to claim 1, further including prunella vulgaris.

4. An herbal formulation for use according to claim 1, further including bombyx mori.

5. An herbal formulation for use according to claim 1, further including phellodendron amurense Rupr.

6. An herbal formulation for use according to claim 1, wherein Camptotheca is in the range of 10% to 90% by weight.

7. An herbal formulation for use according to claim 1, wherein Camptotheca is in the range of 1% to 99% by weight.

8. An herbal formulation for use according to claim 1, wherein poria cocos is in the range of 5% to 20% by weight.

9. An herbal formulation for use according to claim 1, wherein astragalus membranacaus is in the range of 5% to 20% by weight.

10. An herbal formulation for use in lowering the acidity of the human body fluids, comprising:
(a) 75 wt% Camptotheca;
10 wt% poria cocos; and
15 wt% astragalus membranacaus;
(b) 100 wt% Camptotheca: or
(c) 80 wt% Camptotheca; and
20 wt% prunella vulgaris.

## Patentansprüche

1. Pflanzliche Formulierung zur Verwendung bei der Erhöhung der Alkalinität des menschlichen Körpers, umfassend:
Camptotheca;
Poria cocos; und
Astragalus membranacaus.

2. Pflanzliche Formulierung zur Verwendung gemäß Anspruch 1, die ferner Ostrea vivularis gould enthält.

3. Pflanzliche Formulierung zur Verwendung gemäß Anspruch 1, die ferner Prunella vulgaris enthält.

4. Pflanzliche Formulierung zur Verwendung gemäß Anspruch 1, die ferner Bombyx mori enthält.

5. Pflanzliche Formulierung zur Verwendung nach Anspruch 1, die ferner Phellodendron amurense Rupr enthält.

6. Pflanzliche Formulierung zur Verwendung gemäß Anspruch 1, wobei Camptotheca im Bereich von 10 bis 90 Gew.-% liegt.

7. Pflanzliche Formulierung zur Verwendung gemäß Anspruch 1, wobei Camptotheca im Bereich von 1 bis 99 Gew.-% liegt.

8. Pflanzliche Formulierung zur Verwendung gemäß Anspruch 1, wobei Poria cocos im Bereich von 5 bis 20 Gew.-% liegt.

9. Pflanzliche Formulierung zur Verwendung gemäß Anspruch 1, wobei Astragalus membranacaus im Bereich von 5 bis 20 Gew.-% liegt.

10. Pflanzliche Formulierung zur Verwendung bei der Senkung des Säuregehalts der menschlichen Körperflüssigkeiten, umfassend:
(a) 75 Gew.-% Camptotheca;
10 Gew.-% Poria cocos; und
15 Gew.-% Astragalus membranacaus;
(b) 100 Gew.-% Camptotheca: oder
(c) 80 Gew.-% Camptotheca; und
20 Gew.-% Prunella vulgaris.

## Revendications

1. Formulation à base de plantes pour son utilisation dans l'augmentation de l'alcalinité du corps humain comprenant :
du camptotheca ;
du poria cocos ; et
de l'astragalus membranacaus.

2. Formulation à base de plantes pour son utilisation selon la revendication 1, comportant en outre de l'ostrea vivularis gould.

3. Formulation à base de plantes pour son utilisation selon la revendication 1, comportant en outre de la prunella vulgaris.

4. Formulation à base de plantes pour son utilisation selon la revendication 1, comportant en outre du bombyx mori.

5. Formulation à base de plantes pour son utilisation selon la revendication 1, comportant en outre du phellodendron amurense Rupr.

6. Formulation à base de plantes pour son utilisation selon la revendication 1, dans laquelle le camptotheca est présent dans la plage de 10 % à 90 % en poids.

7. Formulation à base de plantes pour son utilisation selon la revendication 1, dans laquelle le camptotheca est présent dans la plage de 1 % à 99 % en poids.

8. Formulation à base de plantes pour son utilisation selon la revendication 1, dans laquelle le poria cocos est présent dans la plage de 5 % à 20 % en poids.

9. Formulation à base de plantes pour son utilisation selon la revendication 1, dans laquelle l'astragalus membranacaus est présent dans la plage de 5 % à 20 % en poids.

10. Formulation à base de plantes pour son utilisation dans l'abaissement de l'acidité des fluides biologiques humains, comprenant :
(a) 75 % en poids de camptotheca ;
10 % en poids de poria cocos ; et
15 % en poids d'astragalus membranacaus ;
(b) 100 % en poids de camptotheca ; ou
(c) 80 % en poids de camptotheca ; et
20 % en poids de prunella vulgaris.
